# EUROPEAN PATENT APPLICATION

(11) **EP 4 218 840 A1**
(43) Date of publication of application: **02.08.2023**
(21) Application number: 21886579.8
(22) Date of filing: 28.09.2021
(51) Int. Cl.: A61L 17/10, A61L 31/04, A61L 31/14, A61B 17/04, C08J 5/18, A61B 17/00

(54) **BIOCOMPATIBLE FILM AND METHOD FOR PRODUCING SAME**

(30) Priority: 26.10.2020 KR 20200139090
(71) Applicant: Innotherapy Inc., Seoul 07282 (KR); Korea Advanced Institute of Science and Technology, Daejeon 34141 (KR)
(72) Inventor: PARK, Shin Hee, Daejeon 34379 (KR); KIM, Soo Mi, Daejeon 34052 (KR); KOH, Mi Young, Seoul 07310 (KR); LEE, Moon Sue, Seoul 07282 (KR); LEE, Haeshin, Daejeon 34094 (KR)
(74) Representative: Prüfer & Partner mbB Patentanwälte · Rechtsanwälte
(86) International application number: PCT/KR2021/013205
(87) International publication number: WO 2022/092585

(57) **Abstract**

The present invention relates to a biocompatible film that exhibits high tensile strength and has biocompatibility and elastic properties, enabling its efficient use in the treatment or protection of human organs. The present invention also relates to a method for producing the biocompatible film.

## Description

### Technical Field

The present invention relates to a biocompatible film with elastic properties as well as high tensile strength and a method for producing the biocompatible film.

### Background Art

Polysaccharide-based polymers such as chitosan, hyaluronic acid, alginic acid, and dextran and protein-based polymers such as collagen and gelatin are commonly known as biopolymers. None of these biopolymers exhibit elastic properties. Specifically, films made of chitosan or hyaluronic acid are not substantially restored after being pulled and films made of collagen or gelatin are not restored at all because they tend to break when pulled.

The surface areas of the stomach, large intestine, small intestine, blood vessels, heart, lungs, and other organs of the human body change periodically due to peristalsis or beating. For effective treatment, materials applicable to these organs should be flexible in response to changes in the surface area of the organs. For example, since the large intestine undergoes a significant change in surface area during peristalsis by the passage of feces, the use of materials with elastic properties capable of withstanding this change is required to ultimately prevent the occurrence of possible leakage after surgery such as intestinal anastomosis. The use of materials with elastic properties capable of withstanding a change in the surface area of blood vessels during blood circulation by the beating of the heart is also required to improve the prognosis of patients after surgery such as angiorrhaphy. Lastly, materials applicable to the lungs are required to have physical properties sufficient to withstand a change in surface area during respiration.

The biocompatibility of materials applied to organs should be more preferentially considered than their flexibility to changes in the surface area of the organs. Biopolymers are known as biocompatible materials, but they are difficult to efficiently apply to organs whose surface area changes due to the lack of elastic properties, as described above. That is, biopolymers for use in the human body after organ surgery are required to have elastic properties as well as biocompatibility, but biopolymers with rubber-like elastic properties have never been reported before.

### Detailed Description of the Invention

### Technical Problems

The present invention is intended to provide a biocompatible film with elastic properties as well as high tensile strength.

The present invention is also intended to provide a method for producing the biocompatible film.

### Means for Solving the Problems

One aspect of the present invention provides a biocompatible film including a first outer layer containing a biopolymer, an inner layer containing a biopolymer, and a first bonding layer disposed between the first outer layer and the inner layer to bond the first outer layer and the inner layer wherein the first bonding layer contains components derived from the first outer layer and components derived from the inner layer.

The components derived from the first outer layer and the components derived from the inner layer may be linked to each other by a physical bond and at least one chemical bond selected from the group consisting of an ionic bond, a hydrogen bond, and a covalent bond.

The biocompatible film of the present invention may further include a second outer layer containing a biopolymer and a second bonding layer disposed between the inner layer and the second outer layer to bond the inner layer and the second outer layer wherein the second bonding layer contains components derived from the inner layer and components derived from the second outer layer.

The components derived from the inner layer and the components derived from the second outer layer may be linked to each other by a physical bond and at least one chemical bond selected from the group consisting of an ionic bond, a hydrogen bond, and a covalent bond.

Each of the biopolymers contained in the first outer layer and the second outer layer may be selected from the group consisting of hyaluronic acid, gelatin, collagen, and mixtures thereof and the biopolymer contained in the inner layer may be selected from the group consisting of chitosan, chitosan introduced with phenol-containing groups, and chitosan introduced with polyhydric phenol-containing groups.

Each of the first outer layer, the second outer layer, and the inner layer may be a nonporous film layer.

The biocompatible film of the present invention may not exhibit elasticity in the absence of a liquid but may exhibit elasticity in the presence of a liquid.

The present invention also provides a method for producing a biocompatible film, including: primarily semi-drying a first outer layer-forming solution containing a biopolymer dissolved therein to form a first outer gel layer; introducing an inner layer-forming solution containing a biopolymer dissolved therein onto the first outer gel layer and secondarily semi-drying the inner layer-forming solution to form an inner gel layer; and introducing a second outer layer-forming solution containing a biopolymer dissolved therein onto the inner gel layer and completely drying the second outer layer-forming solution to form a laminate in which a first outer layer, a first bonding layer, an inner layer, a second bonding layer, and a second outer layer are sequentially formed.

The primary semi-drying may be performed such that the initial weight (100%) of a solvent in the first outer layer-forming solution is reduced by 40 to 90%.

The secondary semi-drying may be performed such that the initial weight (100%) of a solvent in the inner layer-forming solution is reduced by 50 to 95%.

### Effects of the Invention

The biocompatible film of the present invention has high tensile strength and elastic properties as well as biocompatibility, enabling its efficient application to the treatment or protection of human organs whose surface area changes significantly due to peristalsis or beating. Therefore, the present invention can contribute to the reduction of medical expenses for organ-related diseases while reducing complications or mortality after surgery (e.g., intestinal anastomosis, angiorrhaphy, repair of perforated bowel, prevention of bile leakage, pneumonectomy, etc.).

### Brief Description of the Drawings

FIG. 1 is a cross-sectional view of a biocompatible film according to one embodiment of the present invention.
FIG. 2 is a schematic diagram showing bonding relationships between layers constituting a biocompatible film according to one embodiment of the present invention.
FIGS. 3 to 5 are curves showing the proportions of residues introduced per chitosan molecule, which were analyzed in Preparative Examples 1 to 3.
FIG. 6 is a photograph showing a biocompatible film produced in Example 1.
FIG. 7 is a cross-sectional micrograph of a biocompatible film produced in Example 1.
FIGS. 8 to 11 are curves (FIGS. 8 and 9) and images (FIGS. 10 and 11) explaining the results of Experimental Examples 2, 4, 6 and 7, respectively.

### Mode for Carrying out the Invention

It should be understood that the terms and words used in the specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

Since biopolymers are biocompatible enough not to be harmful when absorbed into the human body, they can be usefully applied to the human body. However, commonly known biopolymers do not exhibit rubber-like elastic properties, limiting their application to the treatment or protection of organs whose surface area changes significantly due to peristalsis or beating. Thus, the present inventors have conducted repeated studies and attempts to develop a biocompatible film that exhibits elastic properties as well as biocompatibility, and as a result, have accomplished the present invention. That is, as will be described in detail below, the present invention is directed to a biocompatible stretchable film that exhibits rubber-like elastic properties and is composed only of biopolymers, ensuring high biodegradability. Herein, the elasticity can be defined as the property of the biocompatible film that after being deformed by an external force applied thereto, returns to close to its original shape when the force is removed.

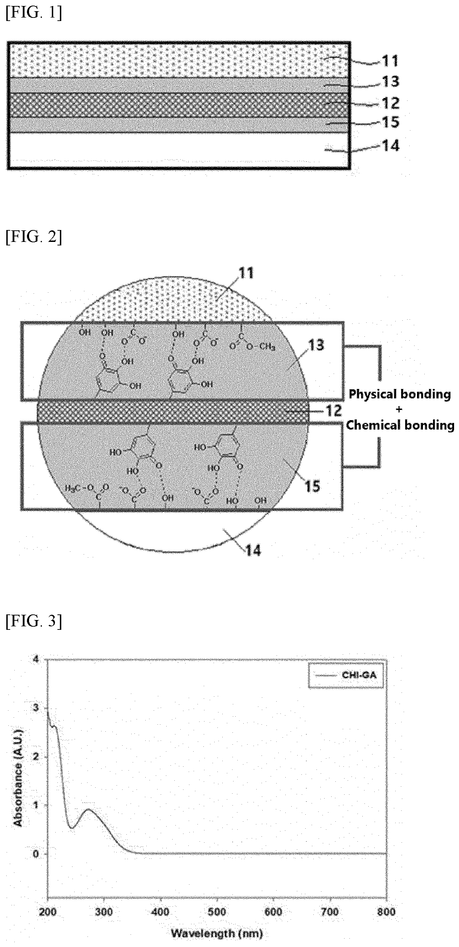

Referring to FIG. 1, a biocompatible film according to one embodiment of the present invention includes a first outer layer 11, an inner layer 12, and a first bonding layer 13.

The first outer layer 11 of the biocompatible film contains a biopolymer (first biopolymer). The biopolymer refers to a polymer that is absorbed *in vivo* by biological degradation or hydrolysis when applied *in vivo.* Specifically, the biopolymer may be a natural biodegradable polymer, a synthetic biodegradable polymer or a nucleic acid. The natural biodegradable polymer may be specifically selected from the group consisting of collagen, fibronectin, fibrin, gelatin, elastin, chitin, chitosan, starch, dextran, alginic acid, hyaluronic acid, cellulose, derivatives thereof, copolymers thereof, and mixtures thereof. The synthetic biodegradable polymer may be specifically selected from the group consisting of poly(lactic acid) (PLA), poly(glycolic acid) (PGA), poly(D,L-lactide-co-glycolide) (PLGA), poly(ε-caprolactone) (PCL), polyhydroxyalkanoate, polyanhydride, polyorthoester, polyamine, derivatives thereof, copolymers thereof, and mixtures thereof.

Specifically, the biopolymer contained in the first outer layer 11 may be an anionically charged one among the above-described biopolymers. The anionic biopolymer is easily bonded to a biopolymer contained in the inner layer 12, which will be described later. This easy bonding enables smooth formation of the first bonding layer 13, through which the first outer layer 11 and the inner layer 12 are bonded to each other, to prevent the first outer layer 11 and the inner layer 12 from separating from each other. As used herein, the term "anionic biopolymer" refers to a biopolymer that is negatively (-) charged when dissolved in a solvent (e.g., water or distilled water). The anionic biopolymer is preferably hyaluronic acid.

The thickness of the first outer layer 11 may be in the range of 1 to 50 µm, specifically 10 to 30 µm. Within this range, the first outer layer exhibits elastic properties, has good wettability and absorbency for fluids, and can be degraded *in vivo* within an appropriate time.

The inner layer 12 of the biocompatible film contains a biopolymer (second biopolymer). The biopolymer refers to a polymer that is absorbed *in vivo* by biological degradation or hydrolysis when applied *in vivo.* Specifically, the biopolymer may be a natural biodegradable polymer, a synthetic biodegradable polymer or a nucleic acid. The natural biodegradable polymer and the synthetic biodegradable polymer are the same as those described above for the first biopolymer and descriptions thereof are omitted.

The biopolymer contained in the inner layer 12 may be a cationically charged one among the above-described biopolymers. The cationic biopolymer is easily bonded to the biopolymer contained in the first outer layer 11 and/or a biopolymer contained in a second outer layer 14, which will be described later. This easy bonding enables smooth formation of the first bonding layer 13, through which the first outer layer 11 and the inner layer 12 are bonded to each other, to prevent the first outer layer 11 and the inner layer 12 from separating from each other and a second bonding layer 15, through which the inner layer 12 and the second outer layer 14 are bonded to each other, to prevent the inner layer 12 and the second outer layer 14 layers from separating from each other. The cationic biopolymer can help hemostasis, for example, because it attracts negatively charged platelets to induce platelet aggregation, achieving hemostasis. As a result, the use of the cationic biopolymer allows effective hemostasis by the inner layer 12. As used herein, the term "cationic biopolymer" refers to a biopolymer that is positively (+) charged when dissolved in a solvent (e.g., water or distilled water). The cationic biopolymer is preferably chitosan.

The biopolymer contained in the inner layer 12 may be a biopolymer introduced with no residues or a biopolymer introduced with phenol-containing groups or polyhydric phenol-containing groups. The introduction of residues in the biopolymer enables the formation of the first bonding layer 13 by which the bonding strength between the first outer layer 11 and the inner layer 12 can be increased and a second bonding layer 15 by which the bonding strength between the inner layer 12 and a second outer layer 14 can be increased, which will be described in detail below.

The phenol-containing groups or polyhydric phenol-containing groups introduced in the biopolymer may be selected from the group consisting of phenol groups, catechol groups, gallol groups, and combinations thereof taking into consideration the bonding strength between the corresponding layers and the elastic properties of the inner layer. The proportion of the phenol-containing groups or polyhydric phenol-containing groups introduced in the cationic biopolymer (the proportion of the phenol-containing groups or polyhydric phenol-containing groups bonded to the cationic biopolymer per unit molecule of the cationic biopolymer) may be 1 to 20 mol%, specifically 5 to 15 mol%.

The thickness of the inner layer 11 may be in the range of 1 to 20 µm, specifically 5 to 15 µm. Within this range, the inner layer exhibits elastic properties and the required flexibility.

The first bonding layer 13 of the biocompatible film is disposed between the first outer layer 11 and the inner layer 12 to bond the first outer layer 11 and the inner layer 12 without separating from each other. The first bonding layer 13 may contain components derived from the first outer layer 11 and the inner layer 12. Specifically, the first bonding layer 13 may be composed of a mixture (*a*) in which components derived from the first outer layer 11 and components derived from the inner layer 12 are mixed with and physically bonded to each other, a polymer or copolymer (b) in which components derived from the first outer layer 11 and components derived from the inner layer 12 are bonded to each other, or a mixture (*a*+*b*) of the components (*a*) and *(b).*

More specifically, the first bonding layer 13 may be composed of components in which components derived from the biopolymer contained in the first outer layer 11 and components derived from the biopolymer contained in the inner layer 12 may be linked to each other by a physical bond and at least one chemical bond selected from the group consisting of an ionic bond, a hydrogen bond, and a covalent bond (see FIG. 2).

The strong bonding between the first outer layer 11 and the inner layer 12 can be maintained by the first bonding layer 13. The formation of the first bonding layer 13 composed of components derived from the first outer layer 11 and the inner layer 12 makes the biocompatible film more biocompatible than the use of a separate component (e.g., an adhesive) that bonds the first outer layer 11 and the inner layer 12.

The thickness of the first bonding layer 13 may be in the range of 0.1 to 5 µm, specifically 0.5 to 3 µm. Within this range, the first bonding layer 13 can exhibit the required flexibility and elastic properties while ensuring high bonding strength between the first outer layer 11 and the inner layer 12.

The biocompatible film may further include a second outer layer 14 and a second bonding layer 15 for the purpose of increasing its tensile strength and elastic properties.

The second outer layer 14 of the biocompatible film contains a biopolymer (third biopolymer). The biopolymer refers to a polymer that is absorbed *in vivo* by biological degradation or hydrolysis when applied *in vivo.* Specifically, the biopolymer may be a natural biodegradable polymer, a synthetic biodegradable polymer or a nucleic acid. The natural biodegradable polymer and the synthetic biodegradable polymer are the same as those described above for the first biopolymer and descriptions thereof are omitted.

The biopolymer contained in the second outer layer 14 may be an anionically charged one among the above-described biopolymers. The anionic biopolymer is easily bonded to the biopolymer contained in the inner layer 12. This easy bonding enables smooth formation of the second bonding layer 15, through which the second outer layer 14 and the inner layer 12 are bonded to each other, to prevent the second outer layer 14 and the inner layer 12 from separating from each other. As used herein, the term "anionic biopolymer" refers to a biopolymer that is negatively (-) charged when dissolved in a solvent (e.g., water or distilled water). The anionic biopolymer is preferably hyaluronic acid.

The second outer layer 14 prevents exposure of the inner layer 12 through the second bonding layer 15, ensuring high tensile strength of the biocompatible film and making the biocompatible film convenient to use. That is, the presence of the biopolymer (for example, chitosan) in the inner layer 12 allows the inner layer to exhibit relatively high adhesion to a counterpart material. If the adherent inner layer 12 is exposed, the biocompatible film adheres to a surgical instrument or a user's hand during use, causing inconvenience in use. The formation of the second outer layer 14 above the inner layer 12 enhances the tensile strength of the biocompatible film and makes the biocompatible film convenient to use.

The thickness of the second outer layer 14 may be in the range of 1 to 50 µm, specifically 10 to 30 µm. Within this range, the second outer layer exhibits the required tensile strength and elastic properties, has good wettability and absorbency for fluids, and can be degraded *in vivo* within an appropriate time.

The second bonding layer 15 of the biocompatible film is disposed between the inner layer 12 and the second outer layer 14 to bond the inner layer 12 and the second outer layer 14 without separating from each other. The second bonding layer 15 may contain components derived from the inner layer 12 and the second outer layer 14. Specifically, the second bonding layer 15 may be composed of a mixture (*a*) in which components derived from the inner layer 12 and components derived from the second outer layer 14 are mixed with and physically bonded to each other, a polymer or copolymer (b) in which components derived from the inner layer 12 and components derived from the second outer layer 14 are bonded to each other, or a mixture (*a*+*b*) of the components (*a*) and *(b).*

More specifically, the second bonding layer 15 may be composed of components in which components derived from the biopolymer contained in the inner layer 12 and components derived from the biopolymer contained in the second outer layer 14 may be linked to each other by a physical bond and at least one chemical bond selected from the group consisting of an ionic bond, a hydrogen bond, and a covalent bond (see FIG. 2).

The strong bonding between the inner layer 12 and the second outer layer 14 can be maintained by the second bonding layer 15. The formation of the second bonding layer 15 composed of components derived from the inner layer 12 and the second outer layer 14 makes the biocompatible film more biocompatible than the use of a separate component (e.g., an adhesive) that bonds the inner layer 12 and the second outer layer 14.

The thickness of the second bonding layer 15 may be in the range of 0.1 to 5 µm, specifically 0.5 to 3 µm. Within this range, the second bonding layer 15 can exhibit the required flexibility and elastic properties while ensuring high bonding strength between the inner layer 12 and the second outer layer 14.

Each of the first outer layer 11, the second outer layer 14, and the inner layer 12 may be a nonporous film layer. That is, the first outer layer 11, the second outer layer 14, the inner layer 12 or all of them may have nonporous film layer structures without pores through which fluids can pass. Both the first bonding layer 13 and the second bonding layer 15 may also be nonporous film layers without pores through which fluids can pass. Due to the nonporous film layer structure of each layer, the biocompatible film is excellent in transportability and storability and has high handleability when applied to a counterpart material (e.g., during a surgical procedure).

The biocompatible film may not exhibit elasticity in the absence (non-existence) of a liquid but may exhibit elasticity in the presence of a liquid. That is, the biocompatible film of the present invention has a specific mechanism in which its solid state without elastic properties is maintained in an environment without a liquid and is changed to a gel state exhibiting elastic properties when placed in an environment containing a liquid. Specifically, the biocompatible film of the present invention is a film that exhibits elastic properties in the presence of a liquid and is thus excellent in transportability, storability, handleability, and applicability. For example, the biocompatible film of the present invention is efficiently applicable to the treatment or protection of human organs whose surface area changes significantly. As an example, the biocompatible film of the present invention may be a film that undergoes a phase transition to exhibit elasticity upon contact with water at room temperature for at least 1 second, specifically for 1 second to 5 minutes.

Specifically, the liquid may be an aqueous solvent such as water, a bodily fluid such as blood, lymph or tissue fluid, or a mixture thereof.

The introduction of the adherent inner layer 12 between the first outer layer 11 and the second outer layer 14, each of which contains a biopolymer, allows stable bonding of the biopolymer molecules, ensuring elastic properties as well as biocompatibility of the biocompatible film. A biopolymer usually exhibits some degree of elasticity at the molecular level. When a biopolymer is dispersed in a solvent to form a biopolymer-containing layer, the molecules are arranged in any random direction, making it difficult to form the biopolymer-containing layer or a biopolymer-containing film that exhibits elastic properties. In contrast, the introduction of the adherent inner layer 12 allows stable bonding of the biopolymer molecules contained in the first outer layer 11 and the biopolymer molecules contained in the second outer layer 14, ensuring elastic properties of the biocompatible film according to the present invention in the presence of a liquid. In addition, the formation of the first bonding layer 13 containing components derived from the first outer layer 11 and the inner layer 12 and the second bonding layer 15 containing components derived from the second outer layer 14 and the inner layer 12 makes the biocompatible film highly biocompatible while ensuring high bonding strengths between the corresponding layers.

As described above, the biocompatible film of the present invention has biocompatibility, elastic properties, and high tensile strength while maintaining high bonding strengths between the constituent layers without separating, enabling its efficient application to the treatment or protection of human organs whose surface area changes significantly. Specifically, the biocompatible film of the present invention can be used to treat or protect sites of intestinal anastomosis, bowel perforation, gastric perforation, gastrectomy, bile leakage, angiorrhaphy, and pneumonectomy. In addition, the biocompatible film of the present invention can be used to treat or protect skin wounds.

The present invention also provides a method for producing the biocompatible film. The method of the present invention is characterized in that the material phases of the first outer layer, the inner layer, and the second outer layer, each of which contains a biopolymer, are controlled such that the layers are firmly bonded to one another. When liquid biopolymers meet each other, they are mixed with each other or aggregate, failing to form a layer. Further, when solid biopolymers meet each other, they cannot be bonded to each other, failing to form a layer. In contrast, according to the method of the present invention, the biopolymers are organically bonded to each other and are controlled to form the corresponding layers (specifically, film layers), which will be described in detail below.

First, a first outer layer-forming solution containing a biopolymer dissolved therein is primarily semi-dried to form a first outer gel layer. The first outer layer-forming solution is prepared by dissolving a biopolymer in a suitable solvent (e.g., water or distilled water). The viscosity of the first outer layer-forming solution may be 1,000 to 100,000 cP (specifically, 10,000 to 50,000 cP). The first outer layer-forming solution is semi-dried rather than completely dried to form a first outer gel layer. The primary semi-drying is performed such that 100% by weight of the solvent in the first outer layer-forming solution is reduced by 40 to 90% (specifically, 50 to 80%).

Next, an inner layer-forming solution containing a biopolymer dissolved therein is introduced onto the first outer gel layer and secondarily semi-dried to form an inner gel layer. The inner layer-forming solution is prepared by dissolving a biopolymer in a suitable solvent (e.g., water or distilled water). The viscosity of the inner layer-forming solution is 1 to 500 cP (specifically, 1 to 200 cP). The inner layer-forming solution is semi-dried rather than completely dried to form an inner gel layer. The semi-drying is performed such that 100% by weight of the solvent in the inner layer-forming solution is reduced by 50 to 95% (specifically, 60 to 90%).

Then, a second outer layer-forming solution containing a biopolymer dissolved therein is introduced onto the inner gel layer and completely dried to form a laminate in which a first outer layer, a first bonding layer, an inner layer, a second bonding layer, and a second outer layer are formed at the same time. The second outer layer-forming solution is prepared by dissolving a biopolymer in a suitable solvent (e.g., water or distilled water). The viscosity of the second outer layer-forming solution is 1,000 to 100,000 cP (specifically, 10,000 to 50,000 cP). The second outer layer-forming solution is completely dried to produce a biocompatible film in which a first outer layer, a first bonding layer, an inner layer, a second bonding layer, and a second outer layer are sequentially bonded to each other. The complete drying is performed such that 100% by weight of the solvent in the second outer layer-forming solution is reduced by at least 95% (specifically, 98 to 100%).

Each of the first outer layer-forming solution, the inner layer-forming solution, and the second outer-layer-forming solution may further include a crosslinking agent for chemical bonding in and between the corresponding layers. The crosslinking agent may be selected from the group consisting of 1,4-butanediol diglycidyl ether (BDDE), 1,2-ethanediol diglycidyl ether (EDDE), diepoxyoctane, and mixtures thereof.

According to the method of the present invention, the material phases are controlled during formation of the corresponding layers such that physical bonding and/or chemical bonding occurs only at the interfaces between the layers to provide a biocompatible film in which a first outer layer, an inner layer, and a second outer layer containing biopolymers (e.g., oppositely charged biopolymers) are firmly bonded to each other.

The present invention will be explained in more detail with reference to the following examples. However, these examples are provided for illustrative purposes and do not serve to limit the scope of the invention. It will be obvious to those skilled in the art that various modifications and changes are possible without departing from the scope and spirit of the invention.

### [Preparative Example 1] Preparation of chitosan introduced with gallol groups

5.2 mmol of chitosan and 6.3 mmol of 1-hydroxybenzotriazole hydrate (HOBt) were added to 100 mL of triple distilled water to prepare a chitosan solution.
5.5 mmol of gallic acid was dissolved in 5 mL of ethyl alcohol and added to the chitosan solution to prepare a chitosan/gallic acid solution.

Next, 6.4 mmol of 1-(3-dimethylaminopropyl)-3ehtylcarbodiimide hydrochloride (EDC) was dissolved in 75 mL of ethyl alcohol and added to the chitosan/gallic acid solution and the reaction was allowed to proceed.

After completion of the reaction, the reaction mixture was dialyzed using a dialysis membrane, followed by freeze-drying to prepare chitosan introduced with gallol groups.

The proportion of gallol groups introduced in the chitosan (bonding rate) was analyzed using a UV-visible spectrophotometer. The results are shown in FIG. 3. The proportion of gallol groups introduced per unit molecule of the chitosan was analyzed to be 9.1 mol%.

### [Preparative Example 2] Preparation of chitosan introduced with phenol groups

5.2 mmol of chitosan and HCl were added to 100 mL of acidic triple distilled water to prepare a chitosan solution.
3.6 mmol of 3-(4-hydroxyphenyl)propionic acid (HPA) was dissolved in 10 mL of ethyl alcohol and added to the chitosan solution to prepare a chitosan/HPA solution.

Next, 3.6 mmol of 1-(3-dimethylaminopropyl)-3ehtylcarbodiimide hydrochloride (EDC) was dissolved in 100 mL of ethyl alcohol and added to the chitosan/HPA solution and the reaction was allowed to proceed.

After completion of the reaction, the reaction mixture was dialyzed using a dialysis membrane, followed by freeze-drying to prepare chitosan introduced with phenol groups.

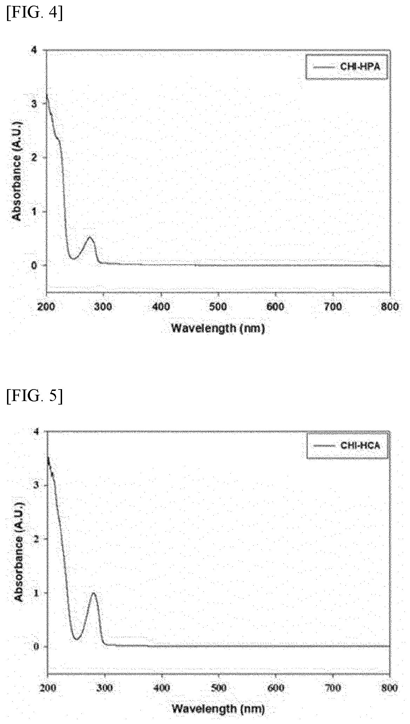

The proportion of phenol groups introduced in the chitosan (bonding rate) was analyzed using a UV-visible spectrophotometer. The results are shown in FIG. 4. The proportion of phenol groups introduced per unit molecule of the chitosan was analyzed to be 6.4 mol%.

### [Preparative Example 3] Preparation of chitosan introduced with catechol groups

5.2 mmol of chitosan and HCl were added to 100 mL of acidic triple distilled water to prepare a chitosan solution.
3.8 mmol of 3,4-dihydroxyhydrocinnamic acid (HCA) was dissolved in 10 mL of ethyl alcohol and added to the chitosan solution to prepare a chitosan/HCA solution.

Next, 3.6 mmol of 1-(3-dimethylaminopropyl)-3ehtylcarbodiimide hydrochloride (EDC) was dissolved in 100 mL of ethyl alcohol and added to the chitosan/HCA solution and the reaction was allowed to proceed.

After completion of the reaction, the reaction mixture was dialyzed using a dialysis membrane, followed by freeze-drying to prepare chitosan introduced with catechol groups.

The proportion of catechol groups introduced in the chitosan (bonding rate) was analyzed using a UV-visible spectrophotometer. The results are shown in FIG. 5. The proportion of catechol groups introduced per unit molecule of the chitosan was analyzed to be 7.8 mol%.

### [Example 1]

Hyaluronic acid was dissolved in triple distilled water to prepare a 1% solution. The solution was put into a PET mold having a size of 10×5 cm and primarily dried until its initial weight was reduced by 65% to form a semi-dried first outer gel layer.

Next, the chitosan introduced with gallol groups prepared in Preparative Example 1 was added to and dissolved in triple distilled water to prepare a 0.66% solution. The solution was introduced onto the first outer gel layer and secondarily dried until its initial weight was reduced by 85% to form a semi-dried inner gel layer.

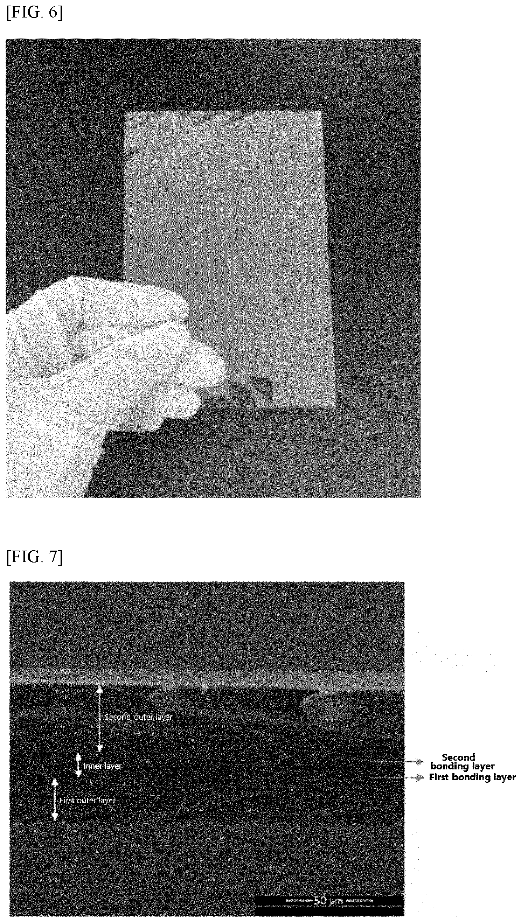

Then, hyaluronic acid was dissolved in triple distilled water to prepare a 1% solution. The solution was introduced onto the inner gel layer and dried until its initial weight was reduced by 96.5%. As a result, a biocompatible film was produced in which a first outer layer, an inner layer, and a second outer layer in completely dry states were formed, a first bonding layer was disposed between the first outer layer and the inner layer, and a second bonding layer was disposed between the inner layer and the second outer layer. Referring to FIG. 6, the biocompatible film was transparent and nonporous.

### [Example 2]

A biocompatible film was produced in the same manner as in Example 1, except that the chitosan introduced with phenol groups prepared in Preparative Example 2 was used instead of the chitosan introduced with gallol groups prepared in Preparative Example 1.

### [Example 3]

A biocompatible film was produced in the same manner as in Example 1, except that the chitosan introduced with catechol groups prepared in Preparative Example 3 was used instead of the chitosan introduced with gallol groups prepared in Preparative Example 1.

### [Example 4]

A biocompatible film was produced in the same manner as in Example 1, except that chitosan introduced with no residues was used instead of the chitosan introduced with gallol groups prepared in Preparative Example 1.

### [Example 5]

A biocompatible film was produced in the same manner as in Example 1, except that gelatin was used instead of hyaluronic acid.

### [Comparative Example 1]

Hyaluronic acid was dissolved in triple distilled water to prepare a 1% solution. The solution was put into a PET mold having a size of 10×5 cm and dried until its initial weight was reduced by 96.5%. As a result, a biocompatible film consisting of a hyaluronic acid monolayer in a completely dry state was produced.

### [Comparative Example 2]

Chitosan was dissolved in triple distilled water to prepare a 2% solution. The solution was put into a PET mold having a size of 10×5 cm and dried until its initial weight was reduced by 97%. As a result, a biocompatible film consisting of a chitosan monolayer in a completely dry state was produced.

### [Comparative Example 3]

The chitosan introduced with gallol groups prepared in Preparative Example 1 was added to triple distilled water and dissolved to prepare a 2% solution. The solution was put into a PET mold having a size of 10×5 cm and dried until its initial weight was reduced by 97%. As a result, a biocompatible film consisting of a chitosan-gallol group monolayer in a completely dry state was produced.

### [Comparative Example 4]

The chitosan introduced with phenol groups prepared in Preparative Example 2 was added to triple distilled water and dissolved to prepare a 2% solution. The solution was put into a PET mold having a size of 10×5 cm and dried until its initial weight was reduced by 97%. As a result, a biocompatible film consisting of a chitosan-phenol group monolayer in a completely dry state was produced.

### [Comparative Example 5]

The chitosan introduced with catechol groups prepared in Preparative Example 3 was added to triple distilled water and dissolved to prepare a 2% solution. The solution was put into a PET mold having a size of 10×5 cm and dried until its initial weight was reduced by 97%. As a result, a biocompatible film consisting of a chitosan-catechol group monolayer in a completely dry state was produced.

### [Experimental Example 1] Cross-section observation

The cross section of the biocompatible film produced in Example 1 was observed by scanning electron microscopy. The results are shown in FIG. 7.

The image of FIG. 7 reveals the formation of the first outer layer, the first bonding layer, the inner layer, the second bonding layer, and the second outer layer in the biocompatible film.

### [Experimental Example 2] Elasticity confirmation

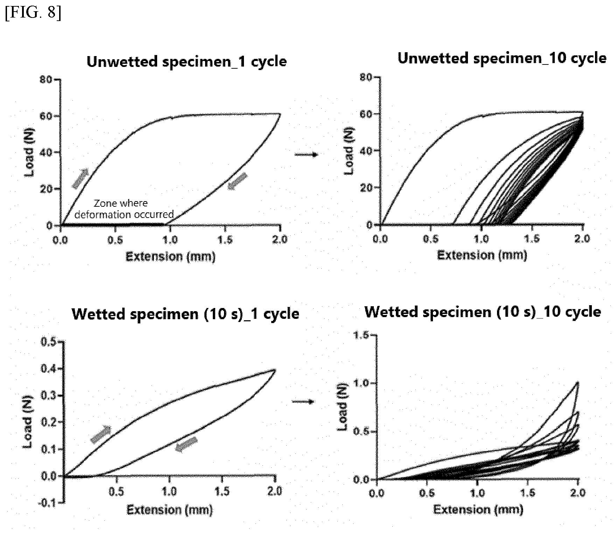

The elasticity of the biocompatible film produced in Example 1 in the presence of a liquid was evaluated by the following procedure using a universal testing machine (UTM). The results are shown as load-extension curves in FIG. 8.

The biocompatible film produced in Example 1 was cut into two rectangular specimens of 10 mm (w) × 50 mm (l). Next, one of the specimens was not wetted with water and the other specimen was wetted water for about 10 sec. Each of the specimens was mounted on the machine and the degrees of elasticity of the specimen in the presence or absence of water were confirmed during repeated 10 cycles of tension and compression.

Referring to the load-extension curves of FIG. 8, the unwetted specimen in a dry state underwent deformation when contracted because it lacked elasticity, whereas the specimen wetted with water for 10 sec showed high elasticity even with a small force.

### [Experimental Example 3] Tensile performance evaluation

The tensile performances of the biocompatible films produced in Examples 1-5 and Comparative Examples 1-5 were evaluated by the following procedure using a universal testing machine. The results are shown in Table 1.

Each of the biocompatible films were cut into dog-bone shaped specimens of 5 mm (w) × 50 mm (l). After the fractured surface of the specimen was wetted with water, the tensile strength of the specimen was measured with a 10 N load cell (a total of three measurements).

As can be seen from the results in Table 1, the inventive biocompatible films had high tensile performances because the biopolymers were bonded to each other in the layers.

**[Table 1]**

| | | Maximum load [N] | Tensile strain at maximum load [%] |
|---|---|---|---|
| Example 1 | Hyaluronic acid/chitosan-gallol/hyaluronic acid (first measurement) | 0.39 | 13.60 |
| | Hyaluronic acid/chitosan-gallol/hyaluronic acid (second measurement) | 0.40 | 17.04 |
| | Hyaluronic acid/chitosan-gallol/hyaluronic acid (third measurement) | 0.55 | 17.52 |
| Example 2 | Hyaluronic acid/chitosan-phenol/hyaluronic acid (first measurement) | 0.31 | 9.54 |
| | Hyaluronic acid/chitosan-phenol/hyaluronic acid (second measurement) | 0.41 | 8.97 |
| | Hyaluronic acid/chitosan-phenol/hyaluronic acid (third measurement) | 0.29 | 9.12 |
| Example 3 | Hyaluronic acid/chitosan-catechol/hyaluronic acid (first measurement) | 1.35 | 9.89 |
| | Hyaluronic acid/chitosan-catechol/hyaluronic acid (second measurement) | 1.75 | 8.84 |
| | Hyaluronic acid/chitosan-catechol/hyaluronic acid (third measurement) | 1.47 | 9.62 |
| Example 4 | Hyaluronic acid/chitosan/hyaluronic acid (first measurement) | 0.87 | 15.34 |
| | Hyaluronic acid/chitosan/hyaluronic acid (second measurement) | 0.62 | 15.40 |
| | Hyaluronic acid/chitosan/hyaluronic acid (third measurement) | 0.68 | 23.94 |
| Example 5 | Gelatin/chitosan-gallol/gelatin (first measurement) | 1.53 | 12.97 |
| | Gelatin/chitosan-gallol/gelatin (second measurement) | 1.45 | 9.47 |
| | Gelatin/chitosan-gallol/gelatin (third measurement) | 2.10 | 17.09 |
| Comparative Example 1 | Hyaluronic acid (first measurement) | 0 | 0 |
| | Hyaluronic acid (second measurement) | 0 | 0 |
| | Hyaluronic acid (third measurement) | 0 | 0 |
| Comparative Example 2 | Chitosan (first measurement) | 10.50 | 5.12 |
| | Chitosan (second measurement) | 6.98 | 4.80 |
| | Chitosan (third measurement) | 8.72 | 5.04 |
| Comparative Example 3 | Chitosan-gallol (first measurement) | 10.43 | 3.19 |
| | Chitosan-gallol (second measurement) | 6.28 | 5.56 |
| | Chitosan-gallol (third measurement) | 7.43 | 3.71 |
| Comparative Example 4 | Chitosan-phenol (first measurement) | 1.61 | 3.96 |
| | Chitosan-phenol (second measurement) | 1.28 | 5.70 |
| | Chitosan-phenol (third measurement) | 1.33 | 5.16 |
| Comparative Example 5 | Chitosan-catechol (first measurement) | 1.95 | 4.40 |
| | Chitosan-catechol (second measurement) | 2.66 | 3.81 |
| | Chitosan-catechol (third measurement) | 3.49 | 4.36 |

### [Experimental Example 4] Elasticity evaluation

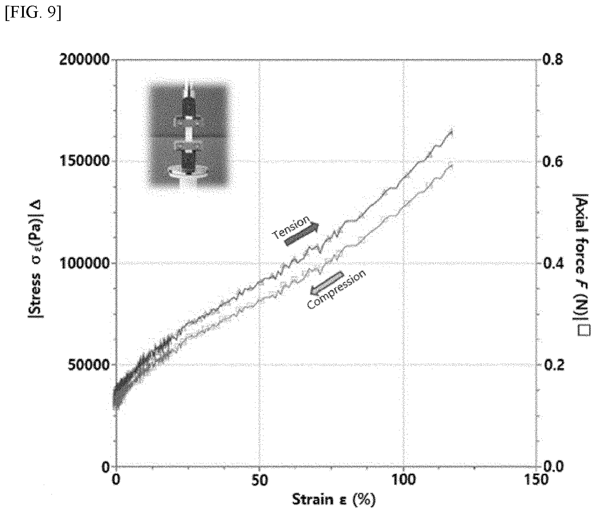

The elasticity of the biocompatible film produced in Example 1 was evaluated by the following procedure using a dynamic mechanical analyzer. The results are shown as stress-strain curves in FIG. 9.

The biocompatible film produced in Example 1 was cut into a rectangular specimen of 10 mm (w) × 50 mm (l). The specimen was wetted with water and mounted on the analyzer. The strains of the specimen were measured while applying forces to the specimen.

As shown in FIG. 9, the stress-strain curve during tension nearly coincided with the stress-strain curve during compression within the error range, demonstrating the elasticity of the film.

### [Experimental Example 5] Adhesive performance evaluation

The adhesive performances of the biocompatible films produced in Example 1 and Comparative Example 1 were evaluated by the following procedure using a bursting tester. The results are shown in Table 2. The evaluation procedure was independently performed in triplicate.

A large intestinal tissue of a pig was perforated using a punch with a diameter of 0.8 cm. Each of the biocompatible films produced in Example 1 and Comparative Example 1 was cut into a square with a size of 2×2 cm and located on the 0.8 cm hole for 1 min. The large intestinal tissue on which the film was located was placed on the diaphragm of the bursting tester and air was blown into the bursting tester to determine a pressure at which the film was separated from the large intestinal tissue.

As can be seen from the results in Table 2, the inventive biocompatible film had a high average adhesive strength of 11.77 hPa.

**[Table 2]**

| | | Pressure (hPa) | Mean | Standard deviation |
|---|---|---|---|---|
| Example 1 | First measurement | 16.07 | 11.77 | 4.34 |
| | Second measurement | 7.39 | | |
| | Third measurement | 11.85 | | |
| Comparative Example 1 | First measurement | 0.64 | 0.21 | 0.37 |
| | Second measurement | 0 | | |
| | Third measurement | 0 | | |

### [Experimental Example 6] Anastomosis performance evaluation - 1

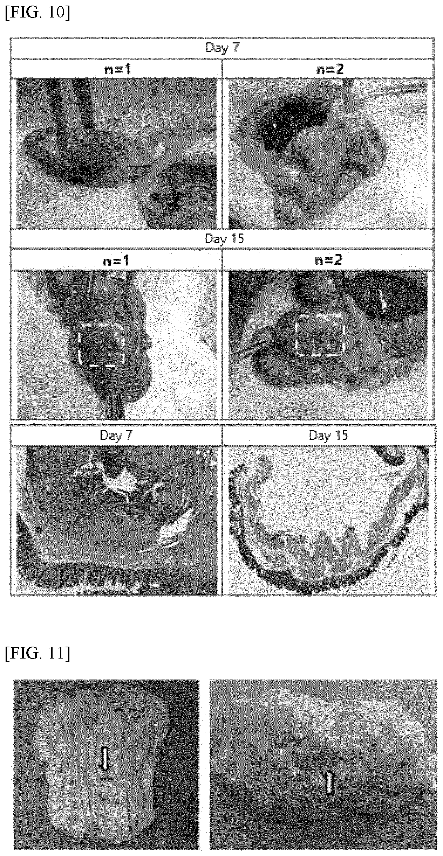

The anastomosis performance of the biocompatible film produced in Example 1 was evaluated using rat models who had undergone cecectomy by the following procedure. The results are shown in FIG. 10. The evaluation procedure was independently performed in duplicate.

A mixture of Zoletil 50 (80 mg/kg) as an anesthetic and Rompun in a 2:1 volume ratio was administered to 9-week-old male rats to induce complete anesthesia. The chest of each anesthetized rat was incised in a straight line and the cecum was exposed to the outside to secure a sufficient field of view. After the exposed cecum was incised about 0.5 cm and sutured with two stitches, a drop of saline was dropped and the biocompatible film of Example 1 was attached thereto. Thereafter, about 1 mL of saline was dropped onto the attached film to prevent the film from adhering to the abdominal wall or other organs. 7 days and 15 days later, it was checked whether the murine cecum was anastomosed. A tissue at the site of anastomosis was excised for histopathology.

Referring to FIG. 10, anastomosis was completed on day 7 and adhesion and fibrosis were drastically reduced on day 15, demonstrating biodegradation of the film.

### [Experimental Example 7] Anastomosis performance evaluation - 2

The anastomosis performance of the biocompatible film produced in Example 1 was evaluated using porcine models who had undergone proctectomy by the following procedure. The results are shown in FIG. 11. The evaluation procedure was independently performed in duplicate.

Female pigs weighing ≥ 30 kg were fasted for one day prior to the experiment and an enema was administered to each animal to evacuate the large intestine as much as possible. Then, an anesthetic was administered to induce complete anesthesia. The abdomen was incised in a straight line and the rectum was exposed to the outside to secure a sufficient field of view. After the exposed rectum was incised ~21 mm straightly, which is a condition in which 100% leakage occurs, the biocompatible film of Example 1 was attached to the incised site without sutures. Thereafter, about 1 mL of saline was dropped onto the attached film to prevent the film from adhering to the abdominal wall or other organs. 15 days later, it was checked whether the porcine rectum was anastomosed.

As can be seen from FIG. 11, the incised rectum was well anastomosed without leakage.

## Claims

1. A biocompatible film comprising a first outer layer containing a biopolymer, an inner layer containing a biopolymer, and a first bonding layer disposed between the first outer layer and the inner layer to bond the first outer layer and the inner layer wherein the first bonding layer contains components derived from the first outer layer and components derived from the inner layer.

2. The biocompatible film according to claim 1, wherein the components derived from the first outer layer and the components derived from the inner layer are linked to each other by a physical bond and at least one chemical bond selected from the group consisting of an ionic bond, a hydrogen bond, and a covalent bond.

3. The biocompatible film according to claim 1, further comprising a second outer layer containing a biopolymer and a second bonding layer disposed between the inner layer and the second outer layer to bond the inner layer and the second outer layer wherein the second bonding layer contains components derived from the inner layer and components derived from the second outer layer.

4. The biocompatible film according to claim 3, wherein the components derived from the inner layer and the components derived from the second outer layer are linked to each other by a physical bond and at least one chemical bond selected from the group consisting of an ionic bond, a hydrogen bond, and a covalent bond.

5. The biocompatible film according to claim 3, wherein each of the biopolymers contained in the first outer layer and the second outer layer is selected from the group consisting of hyaluronic acid, gelatin, collagen, and mixtures thereof and the biopolymer contained in the inner layer is selected from the group consisting of chitosan, chitosan introduced with phenol-containing groups, and chitosan introduced with polyhydric phenol-containing groups.

6. The biocompatible film according to claim 3, wherein each of the first outer layer, the second outer layer, and the inner layer is a nonporous film layer.

7. The biocompatible film according to claim 1, wherein the biocompatible film does not exhibit elasticity in the absence of a liquid but exhibits elasticity in the presence of a liquid.

8. A method for producing a biocompatible film, comprising: primarily semi-drying a first outer layer-forming solution containing a biopolymer dissolved therein to form a first outer gel layer; introducing an inner layer-forming solution containing a biopolymer dissolved therein onto the first outer gel layer and secondarily semi-drying the inner layer-forming solution to form an inner gel layer; and introducing a second outer layer-forming solution containing a biopolymer dissolved therein onto the inner gel layer and completely drying the second outer layer-forming solution to form a laminate in which a first outer layer, a first bonding layer, an inner layer, a second bonding layer, and a second outer layer are sequentially formed.

9. The method according to claim 8, wherein the primary semi-drying is performed such that the initial weight (100%) of a solvent in the first outer layer-forming solution is reduced by 40 to 90%.

10. The method according to claim 8, wherein the secondary semi-drying is performed such that the initial weight (100%) of a solvent in the inner layer-forming solution is reduced by 50 to 95%.
